# EUROPEAN PATENT APPLICATION

(11) **EP 4 129 398 A1**
(43) Date of publication of application: **08.02.2023**
(21) Application number: 22197121.1
(22) Date of filing: 19.11.2020
(51) Int. Cl.: A61N 1/36, A61N 1/05, A61N 1/40, A61N 1/04

(54) **IMPLANTABLE ARRAYS FOR PROVIDING TUMOR TREATING FIELDS**

(30) Priority: 21.11.2019 US 201962938586 P; 30.09.2020 US 202063085658 P
(62) Divisional of application: 20816852.6
(71) Applicant: Novocure GmbH, 6039 Root D4 (CH)
(72) Inventor: CARLSON, Kristen, Concord, 01742 (US)
(74) Representative: Boden, Keith McMurray

(57) **Abstract**

An implantable device can be positioned within a patient proximate to a target site. Electric fields can be generated with the implantable device through the target site at a frequency from about 50 kHz to about 500 kHz. The target site can be a tumor or a peritumoral region. The implantable device can comprise a thin substrate and at least one electrode coupled to the thin substrate or an elongate body and a plurality of electrodes coupled to the elongate body.

## Description

### CROSS-REFERENCE TO RELATED APPLICATION

This application claims priority to and the benefit of U.S. Provisional Application No. 62/938,586, filed November, 21, 2019, and U.S. Provisional Application No. 63/085,658, filed September 30, 2020. Each of the above-identified applications is incorporated herein by reference in its entirety.

### FIELD

This application relates generally to apparatuses and methods for providing tumor treating fields and, in particular, to apparatuses and methods for implanting electrodes within a patient for providing tumor treating fields.

### BACKGROUND

Tumor Treating Fields, or TTFields, are low intensity (e.g., 1-3 V/cm) alternating electrical fields within the intermediate frequency range (100-300 kHz). This non-invasive treatment targets solid tumors and is described in U.S. Pat. No. 7,565,205, which is incorporated herein by reference in its entirety. TTFields disrupt cell division through physical interactions with key molecules during mitosis. TTFields therapy is an approved mono-treatment for recurrent glioblastoma, and an approved combination therapy with chemotherapy for newly diagnosed patients. Conventionally, these electrical fields are induced non-invasively by transducer arrays (i.e., arrays of electrodes) placed directly on the patient's scalp. TTFields also appear to be beneficial for treating tumors in other parts of the body.

### SUMMARY

Described herein, in one aspect, is a method that can comprise positioning an implantable device within a patient proximate to a target site. With the implantable device, electric fields can be generated through the target site at a frequency from about 50 kHz to about 500 kHz. The target site can be a tumor or a peritumoral region. The implantable device can comprise a thin substrate and at least one electrode coupled to the thin substrate.

In other aspects, a method can comprise positioning an implantable device beneath skin of a patient proximate to a target site. The target site can be a tumor or a peritumoral region. The implantable device can comprise an elongate body and a plurality of electrodes coupled to the elongate body. Electric fields can be generated with the implantable device through the target site at a frequency from 50-500 kHz

In further aspects, a method can comprise positioning a first implantable device and a second implantable device within a patient proximate to a target site. The target site can be a tumor or peritumoral region. Each of the first implantable device and the second implantable device can comprise at least one electrode. Electric fields can be generated with the implantable device through the target site at a frequency from 50-500 kHz.

Additional advantages of the invention will be set forth in part in the description that follows, and in part will be obvious from the description, or may be learned by practice of the invention. The advantages of the invention will be realized and attained by means of the elements and combinations particularly pointed out in the appended claims. It is to be understood that both the foregoing general description and the following detailed description are exemplary and explanatory only and are not restrictive of the invention, as claimed.

### BRIEF DESCRIPTION OF THE DRAWINGS

These and other features of the preferred embodiments of the invention will become more apparent in the detailed description in which reference is made to the appended drawings wherein:
FIG. 1 is a block diagram of a system for delivering tumor treating fields using implantable devices and systems as disclosed herein.
FIG. 2 is a schematic diagram of an implantable device disposed within a resection cavity in a cranium of a patient and an additional electrode disposed external to the cranium.
FIG. 3 is a schematic diagram of an implantable device disposed within a resection cavity and an additional electrode disposed external to a peritumoral region.
FIG. 4 is a schematic diagram of a pair of implantable devices disposed on opposing sides of a tumor.
FIG. 5 is a perspective view of a plurality of implantable devices, each device comprising a thin substrate and a plurality of electrodes disposed thereon.
FIG. 6A is a side view of an exemplary implantable device comprising an elongate body. FIG. 6B is a plurality of cross sectional views of the implantable device of FIG. 6A, illustrating different electrode arrangements that can be employed within the implantable device.
FIG. 7A is a side view of an exemplary implantable device comprising an elongate body. FIG. 7B is a cross sectional view of the implantable device of FIG. 7A, illustrating an exemplary electrode arrangement.

### DETAILED DESCRIPTION

The disclosed system and method may be understood more readily by reference to the following detailed description of particular embodiments and the examples included therein and to the Figures and their previous and following description.

It is to be understood that the terminology used herein is for the purpose of describing particular embodiments only and is not intended to limit the scope of the present invention which will be limited only by the appended claims.

It must be noted that as used herein and in the appended claims, the singular forms "a", "an," and "the" include plural references unless the context clearly dictates otherwise. Thus, for example, reference to "an electrode" includes one or more of such electrodes, and so forth.

"Optional" or "optionally" means that the subsequently described event, circumstance, or material may or may not occur or be present, and that the description includes instances where the event, circumstance, or material occurs or is present and instances where it does not occur or is not present.

Ranges may be expressed herein as from "about" one particular value, and/or to "about" another particular value. When such a range is expressed, also specifically contemplated and considered disclosed is the range from the one particular value and/or to the other particular value unless the context specifically indicates otherwise. Similarly, when values are expressed as approximations, by use of the antecedent "about," it will be understood that the particular value forms another, specifically contemplated embodiment that should be considered disclosed unless the context specifically indicates otherwise. It will be further understood that the endpoints of each of the ranges are significant both in relation to the other endpoint, and independently of the other endpoint unless the context specifically indicates otherwise. Finally, it should be understood that all of the individual values and sub-ranges of values contained within an explicitly disclosed range are also specifically contemplated and should be considered disclosed unless the context specifically indicates otherwise. The foregoing applies regardless of whether in particular cases some or all of these embodiments are explicitly disclosed.

Optionally, in some aspects, when values are approximated by use of the antecedents "about," "substantially," "approximately," or "generally," it is contemplated that values within up to 15%, up to 10%, up to 5%, or up to 1% (above or below) of the particularly stated value or characteristic can be included within the scope of those aspects.

Unless defined otherwise, all technical and scientific terms used herein have the same meanings as commonly understood by one of skill in the art to which the disclosed apparatus, system, and method belong. Although any apparatus, systems, and methods and materials similar or equivalent to those described herein can be used in the practice or testing of the present apparatus, system, and method, the particularly useful methods, devices, systems, and materials are as described.

As used herein, the term "patient" refers to a human or animal subject who is in need of treatment using the disclosed systems and devices.

As used herein, the term "electrode" refers to any structure that permits generation of an electric potential, electric current, or electrical field as further disclosed herein. Optionally, an electrode can comprise a transducer. Optionally, an electrode can comprise a non-insulated portion of a conductive element.

Throughout the description and claims of this specification, the word "comprise" and variations of the word, such as "comprising" and "comprises," means "including but not limited to," and is not intended to exclude, for example, other additives, components, integers or steps. In particular, in methods stated as comprising one or more steps or operations it is specifically contemplated that each step comprises what is listed (unless that step includes a limiting term such as "consisting of"), meaning that each step is not intended to exclude, for example, other additives, components, integers or steps that are not listed in the step.

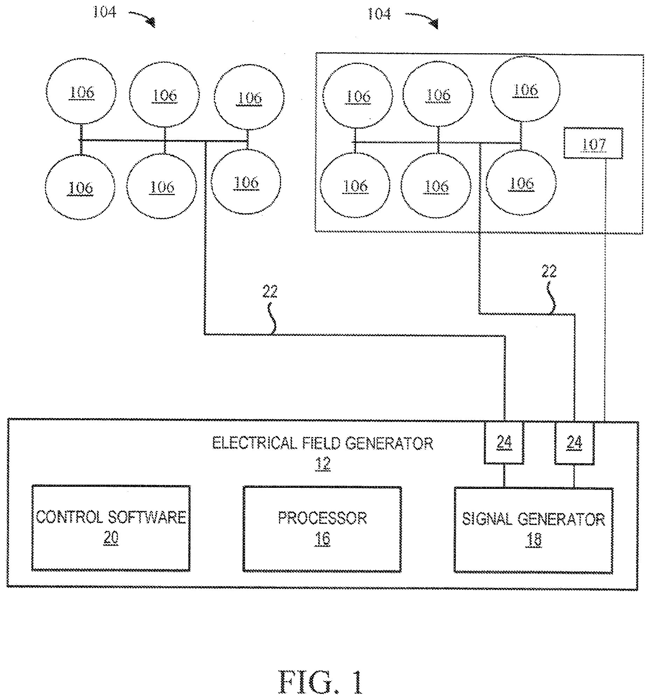

FIG. 1 shows an example apparatus 10 for electrotherapeutic treatment. Generally, the apparatus 10 can be a portable, battery or power supply operated device that produces alternating electrical fields within the body by means of transducer arrays or other electrodes. The apparatus 10 can comprise an electrical field generator 12 and one or more electrode (e.g., transducer) arrays 104, each comprising a plurality of electrodes 106. The apparatus 10 can be configured to generate tumor treating fields (TTFields) (e.g., at 150 kHz) via the electrical field generator 12 and deliver the TTFields to an area of the body through the one or more electrode arrays 104. The electrical field generator 12 can be a battery and/or power supply operated device.

The electrical field generator 12 can comprise a processor 16 in communication with a signal generator 18. The electrical field generator 12 can comprise control software 20 configured for controlling the performance of the processor 16 and the signal generator 18. Although depicted as being within the electrical field generator 12, it is contemplated that the processor 16 and/or control software 20 can be provided separately from the electrical field generator, provided the processor is communicatively coupled to the signal generator and configured to execute the control software.

The signal generator 18 can generate one or more electric signals in the shape of waveforms or trains of pulses. The signal generator 18 can be configured to generate an alternating voltage waveform at frequencies in the range from about 50 KHz to about 500 KHz (preferably from about 100 KHz to about 300 KHz) (e.g., the TTFields). The voltages are such that the electrical field intensity in tissue to be treated is typically in the range of about 0.1 V/cm to about 10 V/cm.

One or more outputs 24 of the electrical field generator 12 can be coupled to one or more conductive leads 22 that are attached at one end thereof to the signal generator 18. The opposite ends of the conductive leads 22 are connected to the one or more electrode arrays 104 that are activated by the electric signals (e.g., waveforms). The conductive leads 22 can comprise standard isolated conductors with a flexible metal shield and can be grounded to prevent the spread of the electrical field generated by the conductive leads 22. The one or more outputs 24 can be operated sequentially. Output parameters of the signal generator 18 can comprise, for example, an intensity of the field, a frequency of the waves (e.g., treatment frequency), a maximum allowable temperature of the one or more electrode arrays 104, and/or combinations thereof. In some aspects, a temperature sensor 107 can be associated with each electrode array 104. Once a temperature sensor measures a temperature above a threshold, current to the electrode array associated with said temperature sensor can be stopped until a second, lower threshold temperature is sensed. The output parameters can be set and/or determined by the control software 20 in conjunction with the processor 16. After determining a desired (e.g., optimal) treatment frequency, the control software 20 can cause the processor 16 to send a control signal to the signal generator 18 that causes the signal generator 18 to output the desired treatment frequency to the one or more electrode arrays 104.

The one or more electrode arrays 104 can be configured in a variety of shapes and positions so as to generate an electrical field of the desired configuration, direction and intensity at a target site (referred to herein also as a "target volume" or a "target region") so as to focus treatment. Optionally, the one or more electrode arrays 104 can be configured to deliver two perpendicular field directions through the volume of interest.

Further disclosure directed to use of such electrotherapeutic systems is provided in U.S. Provisional Patent Application No. 62/942,595, filed December 2, 2019, the entire disclosure of which is hereby incorporated by reference herein.

Although transducers are conventionally positioned externally on the patient, the present disclosure recognizes that there are benefits to positioning electrodes within the body of the patient to provide localized electric fields at the site of a tumor.

Optionally, it is contemplated that the administration of TTFields as disclosed herein can beneficially be combined with temozolomide chemotherapy. Overall survival now extends to over 60 months in some patients when dexamethasone, which was suspected of interfering with tumor-toxic fields effects, is replaced with celecoxib to control tumor-associated inflammation. The transcranial method of delivering tumor-toxic fields has not changed in light of ongoing advances in deep brain stimulation (DBS) and transcranial electric stimulation (TES). The resistivity of the skull is an obstacle to placing therapeutic electric field strength (e.g., at least 2 V/cm, at least 3 v/cm, or at least 4 V/cm), into target tumor sites, and variation in skull thickness can cause a difference in TES efficiency across individuals. Human head finite element modelling (FEM) predicts that surgical craniectomy beneath electrodes that provide tumor-toxic fields can enhance field strength at target tumor sites. For example, using methods disclosed herein, 2-4 V/cm can be reliably delivered to tumor sites using minimally-invasive strip or ribbon electrode arrays in conjunction with a distal transcranial electrode pre- or post-resection. It is contemplated that using frequencies at about 200 kHz (e.g., 100-300 kHz), 1-3 orders of magnitude higher than ion channel time constants, can be too high to stimulate axons *in situ,* thereby avoiding undesirable nervous system side effects. Further, in accordance with embodiments disclosed herein, field strength can be maintained below levels that cause cell damage.

Typically, the body of a cancer patient has an anatomically well-defined mass composed of contiguous cancer cells, or a shell of cancer cells surrounding a 'necrotic' region in which the cells have died due to being starved of nutrients. Such a tumor can be surgically removed ("resected"). The volume of the resection can fill with cerebrospinal fluid in the brain or other body fluid in other body regions, which is electrically-conductive and significantly affects an electric field imposed on the region. Typically, a tumor or resection cavity is surrounded by an anatomically undefined or loosely-defined region containing stray cancer cells, since the extent of stray cancer cells in the vicinity of the tumor is dependent on the tumor cell type, and the highly individualized history, anatomy, immune system, etc. of each patient. The region containing stray, non-contiguous cancer cells is referred to herein as a "peritumoral region."

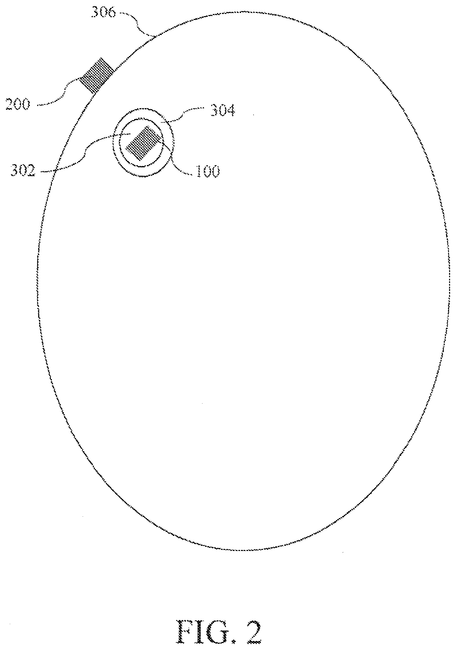

Referring to FIGS. 2-3, in some aspects, a method for treating tumor cells can comprise positioning an implantable device 100 within a patient proximate to a target site (e.g., less than 1 cm from the target site, less than 3 cm of the target site, or within 1-10 cm (or about 1 cm to about 10 cm) of the target site). The proximity to the target site can optionally be as close as possible (without inflicting damage to critical tissue) and no further than a spacing at which the field strength is insufficient to kill the tumor cells within the target site (i.e., a maximum effective distance). The maximum effective distance can be controlled by a number of factors. First, the field strength can be a function of the power provided to the electrodes, and a maximum power threshold can be limited by a temperature threshold. Thus, the implantable device 100 can be positioned proximate to the target site to provide sufficient field strength without surpassing a temperature threshold. For example, it is contemplated that the temperature threshold can be maintained below a temperature threshold at which the patient feels pain (e.g., 41 degrees Celsius) or at a temperature threshold before tissue damage (that can be higher than the latter temperature threshold). It is contemplated that the temperature achieved can be dependent upon the thermal properties of the tissue surrounding the In further aspects, the implantable device 100 can desirably generate sufficient heat (surpassing the temperature threshold at which tissue is damaged) to damage surrounding cells (e.g., ablate surrounding cells). Second, the configuration of the electrode array (further disclosed herein) can determine the shape at which the field emanates from the implantable device 100, thereby affecting the maximum effective distance. Third, the frequency with which the TTFields are applied to the target site can affect the maximum effective distance. Fourth, the geometry of the surrounding tissue and the properties of said surrounding tissue can affect the maximum distance. Accordingly, computational modeling can be used to determine the ideal position of the implantable device with respect to the target site.

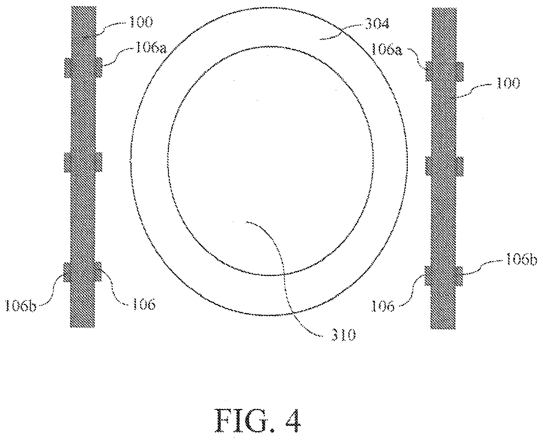

In some aspects, the implantable device 100 can be positioned in a tumor resection cavity 302, within a peritumoral region 304, or adjacent to a tumor or resection cavity, optionally within the peritumoral region 304 or adjacent to the peritumoral region 304. Referring to FIG. 4, in still further aspects, it is contemplated that at least two implantable devices 100 (optionally, three, four, or more implantable devices 100) can be positioned around the target site (e.g., around the resection cavity, or around a tumor 310). For example, optionally, two implantable devices 100 can be positioned on opposing sides or generally opposing sides of the target site. In yet further aspects, it is contemplated that one or more implantable devices 100 can be inserted directly into the tumor 310 or within the peritumoral region 304. For example, optionally, a first implantable device and a second implantable device can be positioned within the peritomoral region 204, and the first and second implatable devices can optionally generate TTFields between each other. According to various aspects, it is contemplated that, with use of TTField treatment, tumor resection can be avoided. Optionally, for generally spherical tumors or cancer cells, a single implantable device 100 can be used, whereas for heterogeneous and non-spherical cancer cells, two (or, optionally, more) implantable devices 100 can be used to generate electric fields that overlap the tumor.

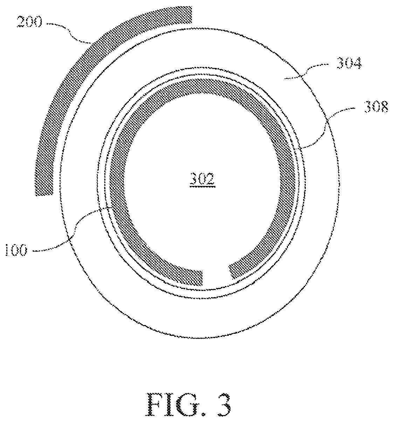

In some aspects, with reference to FIG. 3, one or more electrodes 200 can be positioned outside of the peritumoral region so that the implantable device and the electrode(s) 200 positioned outside of the peritumoral region can generate TTFields therebetween. For example, the implantable device 100 and electrodes 200 can be positioned so that at least a portion of the tumor or peritumoral region lies therebetween (i.e., so that a line extending between the implantable device 100 and the electrodes 200 extends through the tumor or peritumoral region). Optionally, the one or more electrodes 200 can be positioned outside of a cranium 306 (e.g., outside the skin of the patient) or otherwise outside of the body (optionally, outside the skin) of the patient so that at least a portion of the peritumoral region is disposed between the implantable device and the one or more electrodes 200 outside of the peritumoral region. For example, an OPTUNE device (NOVOCURE Gmbh), as is known to those skilled in the art, can be used to position the electrodes 200 against the head of the patient. Optionally, all of the peritumoral region can be disposed between the implantable device and the one or more electrodes 200. In further aspects, in situations where the tumor has not been removed, the one or more electrodes 200 can be positioned so that the tumor is between the implantable device and the one or more electrodes 200. It is contemplated that different patterns of fields can be generated between electrodes based on the arrangement of the electrodes. As disclosed herein, computational modeling can be used to predict the activation patterns of the combined internal and external electrode arrays that are most effective to deliver efficacious field strength to the target region. In general, the target region can be located between the internal and external activated electrodes. However, change of direction of the applied field can be desirable. Accordingly, in some aspects, at least one electrode 200 can be positioned so that an imaginary line between the implantable device and the at least one electrode 200 extends through the target site, and a second pair of electrodes that are skew or orthogonal to the imaginary line between the internal electrodes through the target region to the at least one electrode 200 can be desirable for applying the electric field from a different direction. Thus, modeling can be performed for generic tumor locations, for example, in different quadrants of the tissue. In further aspects, image scans for a given patient can be used to tailor individualized optimal field shaping (e.g., different directional paths of TTFields through the target site) by the various electrodes.

Optionally, in exemplary aspects, the one or more electrodes 200 outside of the peritumoral region can comprise one or more transducer arrays that are configured to apply TTFields through a portion of a brain of a patient. For example, such transducer arrays can be provided as components of an OPTUNE system (NOVOCURE Gmbh) for applying TTFields. In exemplary aspects, it is contemplated that such transducer arrays, when positioned external to the patient (e.g., on the head of the patient), can be used in combination with an implantable device comprising a DBS electrode as further disclosed herein.

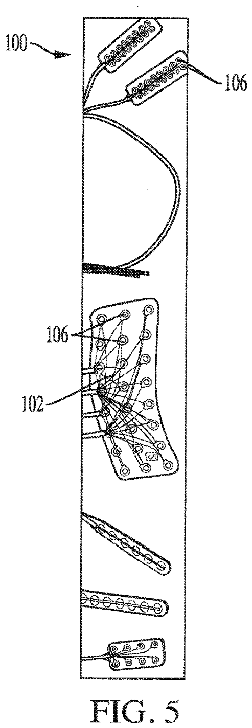

Referring also to FIG. 5, in some aspects, the implantable device can comprise a strip or ribbon electrode assembly comprising a thin substrate 102 with one or more electrodes 106 coupled thereto (optionally, arranged in an array 104). Optionally, the thin substrate 102 can have a thickness of less than 2 mm, such as for example, a thickness from 0.1 mm to 1 mm Exemplary ribbon electrode assemblies that are suitable for use as disclosed herein include subdural grid or strip electrodes manufactured by AD-TECH Medical Instrument Corporation. It is contemplated that the thin substrate 102 can be flexible for select positioning of the electrodes 106 within the resection cavity 302. For example, optionally, the tumor resection cavity can be lined with an anti-bacterial mesh or other surgical material 308. In some aspects, the anti-bacterial mesh or other surgical material 308 can be that which is used in conventional neurosurgery. In some aspects, the anti-bacterial mesh or other surgical material 308 can have negligible electrical resistance or be designed to minimally interfere with, or enhance, the applied electric field. The implantable device 100 can be positioned within the mesh. Optionally, the shape or profile of the implantable device 10 can be bent or otherwise modified to match the contour of the mesh. For example, the implantable device 100 can be pressed against inner surfaces of the mesh 308.

In some aspects, the implantable device 100 can comprise a single electrode 106. In further aspects, the implantable device 100 can comprise a plurality of electrodes 106 that can be arranged in various configurations. For example, in some aspects, the plurality of electrodes 106 can comprise a single row of electrodes that are arranged along an axis. In further aspects, the plurality of electrodes 106 can be arranged on a rectangular grid and can be spaced from each other in equal or unequal spacing. In further aspects, the plurality of electrodes 106 can comprise a plurality or rows of electrodes, with the electrodes within each row being arranged along a respective axis.

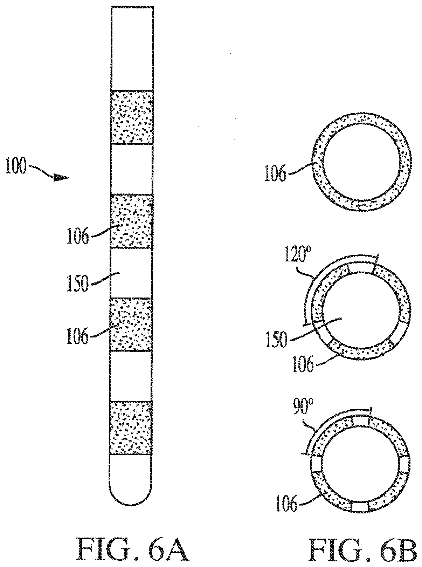

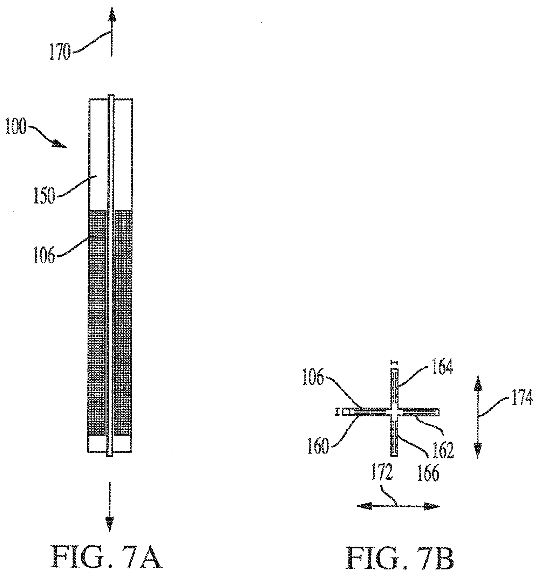

Referring to FIGS. 6A-7B, in further aspects, the implantable device 100 can comprise an elongate body 150 and a plurality of electrodes 106 coupled thereto. The elongate body 150 can optionally be rigid. As shown in FIGS. 6A-6B, the elongate body 150 can optionally define at least one cylindrical surface. In further aspects, as shown in FIGS. 7A-7B, the elongate body can define a cross shape in cross-sections in planes perpendicular to the longitudinal axis. Optionally, in these aspects, the elongate body 150 can comprise a first body portion 160, a second body portion 162, a third body portion 164, and a fourth body portion 166 that converge at the longitudinal axis 170, with the first and second body portions 160, 162 being aligned relative to a first transverse axis 172 that is perpendicular to the longitudinal axis 170 , and with the third and fourth body portions 164, 166 being aligned relative to a second transverse axis 174 that is perpendicular to the longitudinal axis and the first transverse axis. Optionally, the first and second body portions can have equal or substantially equal dimensions relative to the first transverse axis, and the third and fourth body portions can have equal or substantially equal dimensions relative to the second transverse axis. Optionally, the dimensions of the first and second body portions relative to the first transverse axis can be equal or substantially equal to the dimensions of the third and fourth body portions relative to the second transverse axis.

The electrodes for an exemplary implantable device 100 can optionally have uniform dimensions or non-uniform dimensions and can optionally have uniform or non-uniform spacing relative to the longitudinal axis. Optionally, the electrodes can have surface area dimensions ranging from 0.1 mm × 0.1 mm to 1.5mm × 1.15 mm. In further aspects, the electrodes can be larger or smaller, depending on the application. Optionally, the electrodes can be spaced by 0.1 mm or less, by between 0.1 and 0.5 mm, by at least 0.5 mm, by 0.5 mm to 1 mm, or more than 1 mm. The electrodes 106 can optionally be circular, circular profiles projected onto a cylindrical surface, rectangular, rectangular profiles projected onto a cylindrical surface, cylindrical, or any other suitable shape.

In some optional aspects, it is contemplated that the implantable device 100 can be a deep brain stimulation (DBS) probe as is known in the art. Exemplary DBS probes in accordance with embodiments disclosed herein can include MEDTRONIC 3387 DBS probes, MEDTRONIC 3389 DBS probes, ABBOT INFINITY probes, BOSTON SCIENTIFIC probes, DIRECT STNACUTE probes, MEDTRONIC-SAPIENS probes, micro-DBS probes, AD-TEC depth, strip, or ribbon ('Grid') electrodes, AD-TEC subdural electrodes, WISE cortical strips, or DBS probes as described in Anderson, Daria Nesterovich, et al. "Optimized programming algorithm for cylindrical and directional deep brain stimulation electrodes." Journal of neural engineering 15.2 (2018): 026005, which is hereby incorporated by reference herein in its entirety. It is contemplated that the implantable device 100 can have any selected dimensions or any arrangement or distribution of electrodes that is capable of providing electrical stimulation in the manner disclosed herein.

Optionally, it is contemplated that the electrodes 106 can comprise platinum-iridium. In further aspects, it is contemplated that the electrodes can comprise a ceramic. For example, ceramics can have a preferable impedance at certain beneficial frequencies (e.g., 50-500 kHz or 100-300 kHz).

It is contemplated that the electrodes 106 can be activated with a selectable amplitude. Advantageously, the electrodes disclosed herein can be used to generate TTFields in various combinations so that the direction of the field through the target site can be varied. The TTFields can optionally be current- or voltage-controlled. It is contemplated that current-controlled TTFields can achieve more fidelity in a desired waveform (e.g., a rectangular shape) as well as more uniform field string over time as fibrous material (e.g., scar tissue) that is electrically resistive forms around the electrode(s). A current-driven waveform can keep the current and field constant as the electrical resistance changes.

In some aspects, TTFields can be generated between different electrodes 106 of the implantable device 100. In further aspects, TTFields can be generated between electrodes 106 of the implantable device 100 and the one or more electrodes 200. In further aspects, TTFields can be generated between electrodes 106 of two different implantable devices 100.

In some optional aspects, TTFields can be generated at one or more frequencies from 50-500 kHz, optionally, from 100-300 kHz. The field strength through the target areas (e.g., the tumor and/or the peritumoral region) can be at least 2 V/cm, at least 3 V/cm, at least 4 V/cm, or between 2 V/cm and 4 V/cm.

It is contemplated that certain structures, such as microtubules and organelles that can react differently based on the orientation of the field passing therethrough. Accordingly, it is contemplated that the direction of the TTFields can be periodically changed. For example, the activated cathodic and anodic electrodes in an array can be changed periodically to achieve the goal of delivering the most effective field (optionally, the highest field strength) to a given tumor/peritumoral target. In this way, the pattern of activated cathodic and anodic electrodes in an array can be periodically varied to change the direction of the field to optimally deliver the highest field strength to target structures, such as microtubules or organelles, that have different orientations relative to the imposed field due to random cell axis orientations in the target tissues. For example, optionally, the direction of the TTFields can be changed at a frequency of between 0.03 seconds to 0.5 seconds.

In some aspects, changing the direction of the TTFields can comprise switching the polarities of the electrodes inducing the TTFields. For example, a first polarity can be induced between one or more electrodes of the implantable device 100 and the electrode(s) 200 outside the peritumoral region 304 (optionally outside the cranium 308 or otherwise outside of the skin of the patient); and, after a select period, a second polarity, opposite the first polarity, can be induced between the electrode(s) 106 of the implantable device and the electrode(s) 200 outside of the peritumoral region.

In further aspects, the electrodes between which the field is induced can be changed. For example, in some aspects, the field can be induced between at least two electrodes 106 of the implantable device, and, to change direction, the field can be induced between a different combination of electrodes 106 of the implantable device 100. In further aspects, the field can be induced between at least two electrodes 106 of the implantable device, and, to change direction, the field can be switched to being induced between at least one electrode 106 of the implantable device 100 and the electrode(s) 200 outside the peritumoral region. In still further aspects, the field can be induced between a first combination of at least one electrode 106 of the implantable device 100 and the electrode(s) 200, and the change in direction of the field can be caused by changing the electrodes of the implantable device 100 and/or the electrode(s) 200 that are inducing the field. In yet further aspects, the field can be induced between a first combination of at least one electrode 106 of a first implantable device 100 and at least one electrode 106 of a second implantable device 100, and the change in direction of the field can be caused inducing a second combination of at least one electrode 106 of a first implantable device 100 and at least one electrode 106 of a second implantable device 100.

In some aspects, the change in direction of the electric field before and after each direction change can be between 30 and 90 degrees, or between 45 and 90 degrees, or about 90 degrees. In still further aspects, over the course of a single treatment, electric fields can be generated in directions that are angled at at least 30 degrees with respect to each other, at least 45 degrees with respect to each other, or at least 60 degrees with respect to each other. It is contemplated that a mechanism of action of tumor-killing electric fields (i.e., TTFields) is their effect on polarized cell membrane and/or sub-cellular structures. Further, it is contemplated that TTFields can provide significant tumoricidal (tumor-killing) effects when the field is aligned with the cell axis during mitosis, and secondarily, when orthogonal to it. However, it is further contemplated that the tumoricidal (tumor-killing) effects of TTFields can be diminished (or even negligible) when at 45 degrees (or about 45 degrees) to the cell axis. In some aspects, one orthogonal change of direction per second of the applied field can provide a 20% increase in efficacy (in comparison to no change of direction). Since cells are randomly arranged *in vivo,* to achieve the benefit of applying the field aligned with, or orthogonal to, cell axes, changes of direction of the field can be applied over time. It is contemplated that each change of field direction can reduce the variance of field strength to which polarized cell structures are subjected, thereby increasing the minimum field strength to which they are subjected, leading to desirable results. Further, each change of field direction can reduce the maximum field strength necessary to ensure sufficient field strength is delivered to the target.

In some aspects, the processor(s) 16 can be configured to control the polarity induction between electrodes. For example, the processor(s) can alternate induced polarities between two or more electrodes. Accordingly, optionally, in some aspects, the processor(s) 16 can be configured to switch the induced polarity between two or more electrodes so that the electrodes operating as electrodes and anodes and cathodes, respectively, can be reversed after a predetermined period. In some aspects, the processor(s) can repeatedly reverse the induced polarity. In further aspects, the processor(s) can be configured to change which electrodes serve as anode(s) and cathode(s). For example, the processor(s) can cause the electrical field generator 12 (FIG. 1) to induce a field between a first pair of electrodes, and, after certain duration, the processor can cause the electric field generator 12 to induce a field between a second pair of electrodes. Optionally, the processor(s) can execute a protocol stored in a memory that causes the processor to effect a sequential change in polarity and/or electrode combinations in accordance with a stored protocol. For example, a stored protocol can comprise a predetermined sequence of electrode polarity induction for predetermined durations. Such a stored protocol can optionally be customized for generic tumor locations and electrode arrays or for a particular patient given knowledge of tumor location and physical geometry of the patient. In some aspects, for a generic patient, a random sequence can be preferable for effectivity, whereas for a particular patient for which a clinician has knowledge of tumor location and physical geometry, a tailored sequence and/or arrangement can provide optimal tumoricidal results.

### Exemplary Devices and Methods

In some aspects, a single implantable device 100 can be inserted into or proximate to a target area (e.g., a tumor or a tumor resection cavity). The single implantable device 100 can comprise an elongate rigid body 150 and a plurality of electrodes 106 spaced longitudinally along the length of the body. Optionally, the single implantable device 100 can be a DBS probe. It is contemplated that generating electrical fields between electrodes spaced farthest apart can generate a broad (optionally, the broadest possible) field coverage (e.g., through the resection cavity and peritumoral region).

In some aspects, two implantable devices 100 can be inserted into or proximate to (e.g., on opposite sides of) a target area (e.g., a tumor or a tumor resection cavity). In further aspects, it is contemplated that the two implantable device 100 can be positioned within the cavity (e.g., on opposing edges within the resection cavity). The two implantable devices 100 can each comprise an elongate rigid body and a plurality of electrodes 106 spaced longitudinally along the length of the body. Optionally, the two implantable devices 100 can be DBS probes. Different electrodes on each probe can be polarized in sequence to induce electrical fields in different directions. In some aspects, each of the implantable devices 100 can have inner electrodes 106a and outer electrodes 106b, wherein the inner electrodes are relatively closer to the other implantable device than the outer electrodes. In some aspects, TTFields can be generated between respective inner electrodes of each of the two implantable devices to deliver relatively high strength electric fields. In further aspects, TTFields can be generated between respective outer electrodes of each of the two implantable devices to deliver more broadly reaching TTFields (i.e., TTFields propagating further from the electrodes).

In some aspects, ribbon electrode arrays can be placed around the tumor or resection cavity. In some aspects, ribbon electrode arrays can provide smaller and more closely packed electrode arrays than traditional cylindrical DBS probes to more precisely deliver the strongest fields either to the tumor or resection cavity or to the peritumoral region. The electrodes can be activated sequentially around the tumor resection cavity, for example, to deliver strongest doses to the area orthogonal to the electrode faces while delivering a weaker, but still therapeutic dose from a different direction to the areas adjacent to the electrodes.

Optionally, in exemplary aspects, it is contemplated that an implantable device 100 as disclosed herein can be configured for removal from within the patient before the skin of the patient grows over a cavity within which the implantable device is received (e.g., within about 3 to 4 weeks). Optionally, in these aspects, it is contemplated that the cavity within which the implantable device is received can be formed by a craniectomy or other similar procedure. It is further contemplated that after the implantable device is removed, cells from within the patient can be gathered from the implantable device to permit analysis of patient cells that remain on surfaces of the implantable device (e.g., a ribbon or strip electrode as disclosed herein), thereby permitting analysis of tumor cells or cells within the peritumoral region or other target area. Alternatively, in other exemplary aspects, it is contemplated that an implantable device 100 as disclosed herein can be configured to be powered by a battery or other power source positioned external to the patient, thereby providing a long-term implant configuration.

One advantage of using *in situ* TTFields with the external array is the ability to steer the electric field across the tumor/peritumoral region by altering the activated electrodes on the external array. Another advantage of *in situ* tumor-treating fields is the ability to precisely control the field in the vicinity of the target tumor/peritumoral region, thereby avoiding or minimizing collateral damage to nearby, healthy, rapidly-dividing cells. Still further, another advantage is the ability to optimize 1) the static pattern of electrodes at a given point in time, 2) changing field direction in order to impose alignment or orthogonality with cell axes, and 3) changing field direction to attain the largest region of therapeutic dose while minimizing hot spots, which can optionally be characterized by temperature (e.g. 41 degrees Centigrade) to avoid pain and/or by surface current density (such as 30 or 60 microCoulombs per centimeter square) to avoid undesired biochemical reactions at the electrode-tissue interface.

In some aspects, the temperature increase caused by the interaction of current flow through electrically resistive tissue (e.g., "Joule heating") can be a limitation to the maximum voltage, current, and power supplied to device 100. In other aspects, in which deliberate damage to tumor cells proximate to the device is desirable, the implantable device 100 can be used to effect a temperature that kills tissue cells. Accordingly, in some optional aspects, the implantable device 100 can be used for the dual purpose of destroying tumor cells via tumor-killing electric fields as well as killing cells with heat (tissue 'ablation'). A metric such as the Arrhenius equation can be used in computer simulations to predict the 3-dimensional extent of tumor cell ablation, given parameters of voltage, current, or power supplied to device 100. Optionally, the implantable device can comprise a temperature sensor (e.g., a thermocouple or thermistor). In further aspects, a temperature sensor can be positioned proximate to the implantable device for measuring the temperature of the implantable device or the tissue proximate to the implantable device.

The devices and methods disclosed herein can optionally be used to treat glioblastoma or other cancers in the brain. In further optional aspects, the devices and methods disclosed herein can be used to treat cancers or tumor cells in other parts of the body, such as for example, the torso. In these aspects, it is contemplated that the external arrays disclosed herein can be positioned on the skin of a patient while also being in proximity to a tumor or peritumoral region within the body. In exemplary aspects, it is contemplated that the implantable devices disclosed herein can comprise a DBS electrode (e.g., a DBS probe) that is positioned within the torso of a patient.

Although many of the exemplary embodiments disclosed herein are directed to treating brain tumors such as glioblastoma, it should be understood that these examples are not meant to limit the use of embodiments to treating brain tumors. Rather, applications and embodiments of the present disclosure can be used to treat various types of tumors throughout the body of the patient. For example, although various embodiments disclose positioning electrodes outside the skull for treatment of brain tumors, it is contemplated that electrodes can be positioned on various parts of the body depending on the location of the target site. Exemplary target sites outside the brain include the lungs and other internal organs. In exemplary aspects, it is contemplated that electrodes can be positioned outside portions of the torso for treatment of tumors within the lungs or other internal organs, or any other site within the torso where a tumor is identified.

### EXEMPLARY ASPECTS

In view of the described products, systems, and methods and variations thereof, herein below are described certain more particularly described aspects of the invention. These particularly recited aspects should not however be interpreted to have any limiting effect on any different claims containing different or more general teachings described herein, or that the "particular" aspects are somehow limited in some way other than the inherent meanings of the language literally used therein.

Aspect 1: A method comprising: positioning an implantable device within a patient proximate to a target site; and generating, with the implantable device, electric fields through the target site at a frequency from about 50 kHz to about 500 kHz, wherein the target site is a tumor or a peritumoral region, and wherein the implantable device comprises: a thin substrate; and at least one electrode coupled to the thin substrate.

Aspect 2: The method of aspect 1, wherein positioning the implantable device within the patient proximate to the target site comprises positioning the implantable device in a tumor resection cavity.

Aspect 3: The method of aspect 1 or aspect 2, wherein positioning the implantable device within the patient proximate to the target site comprises positioning the implantable device within the tumor or the peritumoral region, wherein the method further comprises: positioning at least one electrode outside of the peritumoral region, wherein generating the electric fields comprises generating electric fields between the implantable device and the at least one electrode outside of the peritumoral region.

Aspect 4: The method of aspect 3, wherein positioning the at least one electrode outside of the peritumoral region comprises positioning the at least one electrode external to skin of the patient so that at least a portion of the peritumoral region is disposed between the implantable device and the at least one electrode outside of the peritumoral region.

Aspect 5: The method of aspects 1, wherein generating the electric fields comprises periodically changing a direction of the electric fields.

Aspect 6: The method of aspect 5, wherein periodically changing the direction of the electric fields comprises changing the direction of the electric field at a frequency of between 0.03 seconds and 0.5 seconds.

Aspect 7: The method of aspect 6, wherein the implantable device comprises a plurality of electrodes, the plurality of electrodes comprising at least a first electrode and a second electrode, wherein changing the direction of the electric field comprises inducing a first polarity at the first electrode and then inducing the first polarity at the second electrode.

Aspect 8: The method of aspect 3, wherein generating the electric fields comprises periodically changing a direction of the electric fields, wherein changing the direction of the electric field comprises: inducing a first polarity between the at least one electrode of the implantable device and the at least one electrode outside of the peritumoral region; and inducing a second polarity, opposite the first polarity, between the at least one electrode of the implantable device and the at least one electrode outside of the peritumoral region.

Aspect 9: The method of aspect 4, wherein generating the electric fields comprises periodically changing a direction of the electric fields, wherein changing the direction of the electric field comprises: inducing a first polarity between at least one electrode of the implantable device and the at least one electrode outside of the peritumoral region; and inducing a second polarity, opposite the first polarity, between the at least one electrode of the implantable device and the at least one electrode outside of the peritumoral region.

Aspect 10: The method of any one of the preceding aspects, wherein the at least one electrode of the implantable device comprises a ceramic.

Aspect 11: The method of any one of the preceding aspects, wherein the target site is within a brain of the patient.

Aspect 12: The method of any one of the preceding aspects, wherein generating, with the implantable device, the electric fields through the target site causes at least a portion of the target site to exceed a threshold temperature sufficient to cause damage to cells of the at least a portion of the target site.

Aspect 13: A method comprising: positioning an implantable device beneath skin of a patient proximate to a target site, wherein the target site is a tumor or a peritumoral region, and wherein the implantable device comprises: an elongate body; and a plurality of electrodes coupled to the elongate body; and generating, with the implantable device, electric fields through the target site at a frequency from 50-500 kHz.

Aspect 14: The method of aspect 13, further comprising: positioning at least one electrode outside the skin of the patient so that at least a portion of the target site is disposed between the implantable device and the at least one electrode, wherein generating, with the implantable device, electric fields through the target site comprises generating electric fields at a frequency from 50-500 kHz between the implantable device and the at least one electrode outside the skin of the patient.

Aspect 15: The method of aspect 13 or aspect 14, wherein the elongate body is rigid.

Aspect 16: The method of any one of aspects 13-15, wherein the plurality of electrodes of the implantable device comprise a ceramic.

Aspect 17: The method of any one of aspects 13-16, wherein generating the electric fields comprises periodically changing a direction of the electric fields.

Aspect 18: A method comprising: positioning a first implantable device and a second implantable device within a patient proximate to a target site, wherein the target site is a tumor or peritumoral region, and wherein each of the first implantable device and the second implantable device comprises at least one electrode; and generating electric fields between the at least one electrode of the first implantable device and the at least one electrode of the second implantable device at a frequency from 50-500 kHz.

Aspect 19: The method of aspect 18, wherein generating the electric fields comprises periodically changing a direction of the electric fields.

Aspect 20: The method of aspect 19, wherein periodically changing the direction of the electric fields comprises changing the direction of the electric fields at a frequency of between 0.03 seconds and 0.5 seconds.

Although the foregoing invention has been described in some detail by way of illustration and example for purposes of clarity of understanding, certain changes and modifications may be practiced within the scope of the appended claims.

### STATEMENTS

Furthermore, the present invention is represented by the following statements of invention.
1. A system, comprising:
   an electrical field generator; and
   an implantable device in electrical communication with the electric field generator and configured for positioning proximate to or within a target site of a patient,
   wherein the electrical field generator is configured to use the implantable device to generate electric fields at a frequency from about 50 kHz to about 500 kHz.
2. The system of statement 1, wherein the implantable device comprises:
   a thin substrate; and
   at least one electrode coupled to the thin substrate.
3. The system of statement 2, wherein the implantable device is configured for positioning in a tumor resection cavity.
4. The system of statement 2, further comprising:
   at least one external electrode in electrical communication with the electrical field generator and configured to be positioned away from the target site, wherein the electrical field generator is configured to generate electric fields between the implantable device and the at least one external electrode.
5. The system of statement 4, wherein the at least one external electrode is configured for positioning external to skin of the patient so that at least a portion of the target site is disposed between the implantable device and the at least one external electrode.
6. The system of statement 2, wherein the electrical field generator is configured to periodically change a direction of the electric fields.
7. The system of statement 6, wherein the electrical field generator is configured to change the direction of an electric field at a frequency of between 0.03 seconds and 0.5 seconds.
8. The system of statement 7, wherein the at least one electrode of the implantable device comprises a plurality of electrodes, the plurality of electrodes comprising at least a first electrode and a second electrode, and the electrical field generator is configured to change the direction of the electric field by inducing a first polarity at the first electrode and then inducing the first polarity at the second electrode.
9. The system of statement 4, wherein the electrical field generator is configured to periodically change a direction of the electric fields by:
   inducing a first polarity between at least one electrode of the implantable device and the at least one external electrode; and
   inducing a second polarity, opposite the first polarity, between the at least one electrode of the implantable device and the at least one external electrode.
10. The system of statement 5, wherein the electrical field generator is configured to periodically change a direction of the electric fields by:
   inducing a first polarity between at least one electrode of the implantable device and the at least one external electrode; and
   inducing a second polarity, opposite the first polarity, between the at least one electrode of the implantable device and the at least one external electrode.
11. The system of statement 2, wherein the at least one electrode of the implantable device comprises a ceramic.
12. The system of statement 2, wherein the implantable device is configured for positioning within a brain of the patient.
13. The system of statement 2, wherein the electrical field generator is configured to generate electric fields through the target site that cause at least a portion of the target site to exceed a threshold temperature sufficient to cause damage to cells of the at least a portion of the target site.
14. The system of statement 1, wherein the implantable device comprises:
   an elongate body; and
   a plurality of electrodes coupled to the elongate body.
15. The system of statement 14, further comprising:
   at least one external electrode in electrical communication with the electrical field generator and configured to be positioned away from the target site, wherein the electrical field generator is configured to generate electric fields between the implantable device and the at least one external electrode.
16. The system of statement 14, wherein the elongate body is rigid.
17. The system of statement 14, wherein the plurality of electrodes of the implantable device comprise a ceramic.
18. The system of statement 14, wherein the electrical field generator is configured to periodically change a direction of the electric fields.
19. The system of statement 1, wherein the implantable device is a first implantable device, the system comprises a second implantable device, the second implantable device comprising at least one electrode, and the electrical field generator is configured to generate electric fields between the at least one electrode of the first implantable device and the at least one electrode of the second implantable device.
20. The system of statement 19, wherein the electrical field generator is configured to periodically change a direction of the electric fields.
21. The system of statement 20, wherein the electrical field generator is configured to change the direction of the electric fields at a frequency of between 0.03 seconds and 0.5 seconds.
22. A method, comprising:
   positioning an implantable device within a patient proximate to a target site; and generating, with the implantable device, electric fields through the target site at a frequency from about 50 kHz to about 500 kHz, wherein the target site is a tumor or a peritumoral region.
23. The method of statement 22, wherein the implantable device comprises:
   a thin substrate; and
   at least one electrode coupled to the thin substrate.
24. The method of statement 23, wherein positioning the implantable device within the patient proximate to the target site comprises positioning the implantable device in a tumor resection cavity.
25. The method of statement 23, wherein positioning the implantable device within the patient proximate to the target site comprises positioning the implantable device within the tumor or the peritumoral region, and the method further comprises:
   positioning at least one electrode outside of the peritumoral region, wherein generating the electric fields comprises generating electric fields between the implantable device and the at least one electrode outside of the peritumoral region.
26. The method of statement 25, wherein positioning the at least one electrode outside of the peritumoral region comprises positioning the at least one electrode external to skin of the patient so that at least a portion of the peritumoral region is disposed between the implantable device and the at least one electrode outside of the peritumoral region.
27. The method of statement 23, wherein generating the electric fields comprises periodically changing a direction of the electric fields.
28. The method of statement 27, wherein periodically changing the direction of the electric fields comprises changing the direction of the electric field at a frequency of between 0.03 seconds and 0.5 seconds.
29. The method of statement 28, wherein the implantable device comprises a plurality of electrodes, the plurality of electrodes comprising at least a first electrode and a second electrode, and changing the direction of the electric field comprises inducing a first polarity at the first electrode and then inducing the first polarity at the second electrode.
30. The method of statement 25, wherein generating the electric fields comprises periodically changing a direction of the electric fields, and changing the direction of the electric field comprises:
   inducing a first polarity between the at least one electrode of the implantable device and the at least one electrode outside of the peritumoral region; and
   inducing a second polarity, opposite the first polarity, between the at least one electrode of the implantable device and the at least one electrode outside of the peritumoral region.
31. The method of statement 26, wherein generating the electric fields comprises periodically changing a direction of the electric fields, and changing the direction of the electric field comprises:
   inducing a first polarity between at least one electrode of the implantable device and the at least one electrode outside of the peritumoral region; and
   inducing a second polarity, opposite the first polarity, between the at least one electrode of the implantable device and the at least one electrode outside of the peritumoral region.
32. The method of statement 23, wherein the at least one electrode of the implantable device comprises a ceramic.
33. The method of statement 23, wherein the target site is within a brain of the patient.
34. The method of statement 23, wherein generating, with the implantable device, the electric fields through the target site causes at least a portion of the target site to exceed a threshold temperature sufficient to cause damage to cells of the at least a portion of the target site.
35. The method of statement 22, wherein the implantable device comprises:
   an elongate body; and
   a plurality of electrodes coupled to the elongate body.
36. The method of statement 35, further comprising:
   positioning at least one electrode outside the skin of the patient so that at least a portion of the target site is disposed between the implantable device and the at least one electrode, wherein generating, with the implantable device, electric fields through the target site comprises generating electric fields at a frequency from 50-500 kHz between the implantable device and the at least one electrode outside the skin of the patient.
37. The method of statement 35, wherein the elongate body is rigid.
38. The method of statement 35, wherein the plurality of electrodes of the implantable device comprise a ceramic.
39. The method of statement 35, wherein generating the electric fields comprises periodically changing a direction of the electric fields.
40. The method of statement 22, wherein the implantable device is a first implantable device, the method further comprising:
   positioning a second implantable device within the patient proximate to the target site, wherein the second implantable device comprises at least one electrode, and generating, with the implantable device, electric fields through the target site at a frequency from about 50 kHz to about 500 kHz comprises generating electric fields between the at least one electrode of the first implantable device and the at least one electrode of the second implantable device.
41. The method of statement 40, wherein generating the electric fields comprises periodically changing a direction of the electric fields.
42. The method of statement 41, wherein periodically changing the direction of the electric fields comprises changing the direction of the electric fields at a frequency of between 0.03 seconds and 0.5 seconds.

## Claims

1. A system, comprising:
an electric field generator; and
an implantable device in electrical communication with the field generator and configured for positioning proximate to or within a target site of a patient; wherein the field generator is configured to use the implantable device to generate electric fields at a frequency of from about 50 kHz to about 500 kHz, and periodically change a direction of the electric fields at a frequency of between 0.03 seconds and 0.5 seconds.

2. The system of claim 1, wherein the implantable device comprises a plurality of electrodes, the plurality of electrodes comprising at least first and second electrodes, and the field generator is configured to change the direction of the electric fields by inducing a first polarity at the first electrode and then inducing the first polarity at the second electrode.

3. The system of claim 1, further comprising:
at least one external electrode in electrical communication with the field generator and configured to be positioned away from the target site, wherein the field generator is configured to generate electric fields between the implantable device and the at least one external electrode.

4. The system of claim 3, wherein the at least one external electrode is configured for positioning external to skin of the patient so that at least a portion of the target site is disposed between the implantable device and the at least one external electrode.

5. The system of claim 3, wherein the field generator is configured periodically to change a direction of the electric fields by inducing a first polarity between at least one electrode of the implantable device and the at least one external electrode, and inducing a second polarity, opposite the first polarity, between the at least one electrode of the implantable device and the at least one external electrode.

6. The system of claim 1, wherein the implantable device is a first implantable device, and further comprising:
a second implantable device, wherein the field generator is configured to generate electric fields between at least one electrode of the first implantable device and at least one electrode of the second implantable device.

7. The system of claim 1, wherein the field generator is configured to generate electric fields through the target site that cause at least a portion of the target site to exceed a threshold temperature sufficient to cause damage to cells of the at least a portion of the target site.

8. The system of claim 1, wherein the implantable device comprises a thin substrate and at least one electrode coupled to the substrate.

9. The system of claim 1, wherein the implantable device comprises an elongate body and a plurality of electrodes coupled to the elongate body.

10. The system of claim 9, wherein the elongate body is rigid.

11. The system of claim 9, wherein the elongate body has a cross shape with first to fourth body portions which converge on an axis.

12. The system of claim 9, wherein the electrodes are disposed in spaced location along a length of the elongate body.

13. The system of claim 9, wherein the electrodes are disposed in spaced location about a circumference of the elongate body.

14. The system of claim 1, wherein the implantable device is configured for positioning within a brain of the patient.

15. The system of claim 1, wherein the implantable device is configured for positioning in a tumor resection cavity of the patient.
